# EUROPEAN PATENT SPECIFICATION

(11) **EP 2 067 453 B1**
(45) Date of publication and mention of the grant of the patent: **10.08.2011**
(21) Application number: 07023687.2
(22) Date of filing: 06.12.2007
(51) Int. Cl.: A61F 2/14

(54) **Keratoprosthesis**
Hornhautprothese
Prostokératoplastie

(43) Date of publication of application: 10.06.2009
(73) Proprietor: MIRO GmbH, 81241 München (DE)
(72) Inventor: Müller-Lierheim, Wolfgang G.K., 81243 München (DE)
(74) Representative: Eisenführ, Speiser & Partner

(56) References cited:
- EP-A- 0 956 834
- US-A- 5 130 160
- US-A1- 2007 168 025

## Description

The invention concerns a keratoprosthetis having a central optical part connected to a peripheral haptic part both made of hydrophobic material.

Opacification of the cornea of the human eye results in the loss of vision and finally blindness unless corrected by a corneal transplant. Conditions that may require corneal transplants include Keratoconus (a local steepening of the curvature of the cornea) if it cannot be corrected by special contact lenses, hereditary corneal failure (e.g. Fuchs endothelial dystrophy), extensive scarring and/or vascularization after infections (e.g. Herpes and trachoma) or penetrating injury, and corneal opacification after eye surgery including refractive surgery (LASIK). The most frequent surgical technology to restore vision is the replacement of the cornea by a human donor cornea in a penetrating keratoplasty. In this procedure which was first performed by Zirm in 1906 a circular part of the damaged cornea is removed and replaced by the respective part of a cadaver cornea which is sutured into the host cornea. In developed countries corneal transplantion using donor cornea provided by a network of corneal banks are the most common and successful operations in transplant surgery. More than 40.000 keratoplasties per year are performed in Europe and the United States each, with a continuous increase in recent years.

Recipients of corneal transplants generally require long-term local therapy with antibiotics, antiinflammatory and anti-rejection drugs. Depending on a number of circumstances the success rate (i.e. clear transplant six months after surgery) varies from more than 90 to less than 50 percent. Low success rates are associated with dry eyes, Herpes keratitis, corneal vascularization, recurring uveitis, acid burns, and traumatic anatomic structures of the anterior eye. The prognosis of keratoplasty also depends on the quality of the donor cornea including its storage and transport. There is a lack of donor corneas resulting in long waiting lists of patients in developed countries. Due to the lack of infrastructure there are no corneal banks in developing countries resulting in millions of treatable blind people in these countries.

The idea to replace the human cornea by alloplastic material dates back more than 200 years. In 1789 the French ophthalmologist Pellier de Quengsy proposed the implantation of a glass plate surrounded by a silver ring into the human cornea. In 1853 Nussbaum performed during his doctoral thesis in Munich experiments on rabbit eyes with a reported implant survival time of seven months. Six years later the Swiss surgeon Heusser implanted a glass plate into the cornea of a 19 year old who lost the implant after only three months. Between 1877 and 1887 von Hippel implanted seven corneal implants in human eyes with a maximum survival time of 12 months. Due to this experience, the development focused on the combination of clear optics with surrounding haptics, and in 1900 Salzer reported on the survival of 2,5 years of a quartz optic with horn haptic. The development of alloplastic keratoplasties stopped when Zirm reported on successful kerastoplasty with homologues implants in 1906.

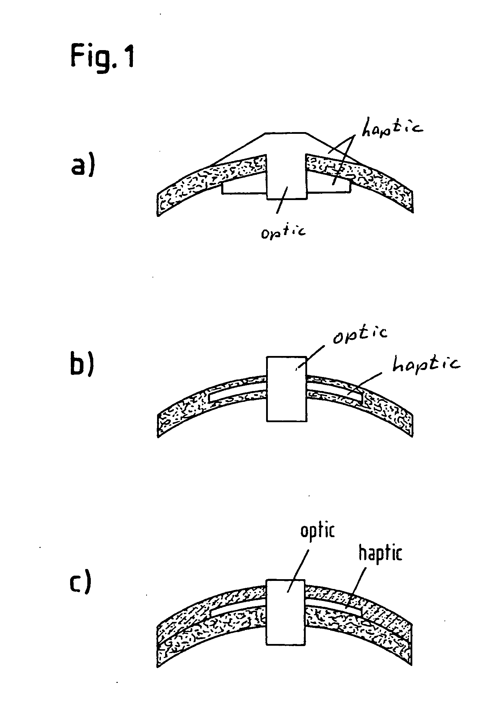

It was not before 1940 when Wünsche re-initiated experiments with alloplastic material: Polymethylmethacrylate (PMMA). The publications of Ridley and Roper-Hall about the intraocular biocompatibility of PMMA encouraged the use of this material in keratoplasty. Best results were achieved with penetrating implants with an optic made of PMMA and various designs of circular haptic intended to fix the optic on or in the cornea (K. Hille, Keratoprothesen - Historischer Überblick, Materialien und Stand der gegenwärtigen Forschung, Ophthalmologe 99, 513-522, 2002). Figure 1 (a) shows an epiendocomeal ('nut-and-bolt'), (b) an endostromal, and (c) an epicorneal keratoprosthesis. The epicorneal prosthesis is most frequently covered with an oral mucous transplant.

The long-term results with these PMMA keratoprostheses were generally disappointing. Complications included the melting of tissues next to the haptic, leakage resulting in infections, aseptic inflammation, and epithelialization of the surface of the optic resulting in opacification and rejection. Moreover, there was a high incidence of vitreous inflammation and glaucoma.

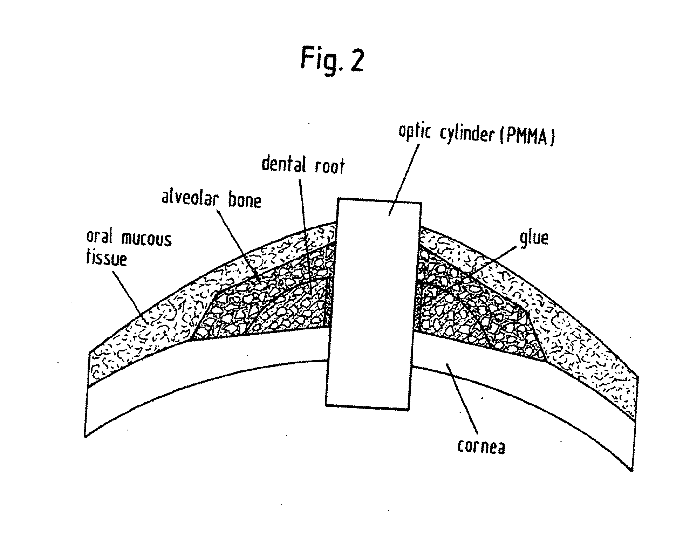

In the early sixties Strampelli developed a keratoprothesis using biological material for the haptic. For his 'osteoodontokeratoprothesis' (OOKP) Strampelli removed a single-rooted tooth together with the surrounding alveolar bone from the patient's mouth and used this for preparing the haptic. He cut a precision hole into this haptic and fixed a long cylindrical optic made of PMMA into this hole by glue (Figure 2). It turned out that the rejection rate of Strampelli's implant is rather low, and it is, therefore, by leading ophthalmosurgeons since 40 years considered as the ultima ratio for patients where a keratoplasty with a donor cornea is impossible (K. Hille, Keratoprothesen - Klinische Aspekte, Ophthalmologe 99, 523-531, 2002). However, this implant has a number of shortcomings. It requires three surgical operations. The patient still needs to have a vital single root tooth available for removal together with the surrounding alveolar bone. The implant needs to undergo a three months conditioning phase by placing it in a subcutaneous bag at the lower lid. The cylindrical optic penetrates deeply into the anterior chamber of the eye. The iris and lens have to be removed in order to prevent the growth of retroprosthetic membranes and the development of secondary glaucoma. Due to the size and rigidity of the implant conventional screening of glaucoma is impossible leaving the risk of loss of vision due to glaucoma. And the field of vision is narrow due to the long narrow cylindrical optic. On the other hand this implant has turned out to be tolerated over many years even by patients with dry eyes and a poor prognosis for corneal transplants.

For patients without a single-rooted tooth Pintucci modified Strampelli's keratoprosthesis design in 1979 by replacing the dental root with alveolar bone by a soft, pliable Polyethyleneterephthalat (Dacron) tissue made of fabrics. Just like the Strampelli implant the Dacron felt of Pintucci's keratoprosthesis requires preconditioning to allow the ingrowth of tissue in the Dacron felt. The keratoprosthesis with the preconditioned Dacron haptic is then implanted by covering the haptic on top of the cornea with oral mucous tissue and suturing it to the cornea. Oral mucous tissue is preferably used because of its mechanical strength, strong vascularization and fast cellular tum-over.

Recent efforts to develop improved keratoprostheses focus on the use of flexible, biocompatible, porous haptic materials for example from Polytetrafluorethylene (PTFE), Polyethyleneterephthalat (Dacron) or poly-2-Hydroyethylmethacrylate (pHEMA). However the long-term integration of these haptic materials into the body tissues was so far not satisfactory. Also attempts to replace the rigid optic with flexible silicone or poly-2-Hydroyethylmethacrylate (pHEMA) were not very successful.

Out of the currently available keratoprostheses the Strampelli design has the best long-term clinical success, followed by the Pintucci design. Both keratoprosthesis designs require a long optical cylinder with a small diameter in order to prevent the growth of retroprosthetic membranes. This means that both the iris and lens have to be removed and the patient will have a small visual field.

US 2007/168025 A1 describes in conformity with the preamble of claim 1 a keratoprosthesis having a central optical part tightly connected to peripheral haptic part both made of hydrophobic polymers, whereas the chemical nature of the surface oriented to the anterior chamber of the eye is such that it does not support fibrin adherence and membrane formation. The textured haptic part is configured to promote the adherence of cells and eventually vascular ingrowth. The optical part has the configuration of a lens which is adapted to the form of the cornea of the eye. The surface of the optical part oriented toward the eyelid is formed by the hydrophobic polymer.

EP-A-0 956 834 discloses a keratoprosthesis having an optical part in form of a lens which is adapted to the form of the cornea, whereas the surfaces of the optical part are coated by polyvinylpyrrolidone.

Object of the invention is to provide a keratoprosthesis which has an improved visual field and can be implanted easily.

The object is solved by a keratoprosthesis having the features of claim 1.

Rendering the chemical nature of the posterior surface of the optic part such that the formation of a retroprosthetic membrane is prevented allows to significantly reduce the length of the optical cylinder thus enlarging the visual field and allowing the patient's iris and lens to remain in place.

The intraocular pressure exercised on the unsupported optical part of the keratoprosthesis must be born by the haptic part attached to the remaining cornea. The force exercised by the intraocular pressure is proportional to the cross section of the optic, that means proportional to the square of the optic diameter. Therefore, the optic diameter shall be as small as reasonably possible, whereas the overall diameter of the keratoprosthesis should be large in order to distribute the forces across a large haptic area.

A rigid haptic has the disadvantage of not following the movement of corneal tissues thus causing local mechanical stress. A flexible haptic will follow the movement of the surrounding corneal tissue and prevent local stress. According to the invention the rigidity of the haptic material is similar to the corneal tissue, i.e. mimic the material. If the haptic material is too soft the haptic will exercise strong radial forces Fᵣ not evenly distributed over the corneal tissue and cause shear stress.

Both optic and haptic material is hydrophobic, i.e. absorb less than 5 percent water in order to avoid interaction with eye medications and dimensional changes due to changes in hydration. Preferrably both optic and haptic are made of the same flexible optically clear polymer. Alternatively the flexible haptic polymer can be polymerized as an interpenetrating network to the optic polymer which then may be rigid.

The front portion of the optic not in contact with corneal tissue shall preferably be coated by a hydrophilic layer adsorbing water, enable a smooth gliding of the eye lid and support the spreading of artificial tear when instilled in the eye.

The haptic shall enable the anchoring and ingrowth of surrounding tissue including vascularization. In order to enable this the topograghy of surface can be textured with ridges, groves, pillars, cylindric holes, pores, mesh structure, spikes or similar. In order to maximize cellular attachment and minimize inflammatory response the surfaces of the keratoprosthesis in contact with tissue has preferably a cell adhesive biochemical coating such as fibronecting and/or use the topography-associated surface free energy to promote cell adherence. Pores or holes preferably penetrate the haptic in order to allow vascularization.

By creating keratoprostheses with such biomimetic characteristics the most serious drawbacks of the known designs could be overcome, not requiring a several step surgical procedure with preconditioning of the haptic part.

### Embodiments of the invention

### Coating of PMMA with Heparin

The surface of clinical quality Polymethylmethacrylate (PMMA) platelets was saponified by incubation in 3 M Sodium hydroxide at 70°C for 24 hours. Thereby a negatively charged surface was created. After washing the activated PMMA platelets with Sodium carbonate buffer solution at pH 9 the surface was coated with Polyethyleneimine (PEI) by ionic bonding. The successful coating with PEI was verified by staining with Eosin red solution. Sodium heparine was activated by Sodium nitrite solution. The PEI coated PMMA platelets were incubated with activated Heparin solution. Subsequently the binding of the activated Heparin to the PEI coated PMMA surface was initiated by incubation with Sodium borohydride solution. The successful Heparin binding was verified by staining with Toluidine blue solution.

*In vitro* cell adherence tests showed that the heparinized PMMA surfaces strongly inhibited the adherence of human fibroblasts as compared to untreated PMMA surfaces, confirming the potential for preventing retroprosthetic membrane formation on the optical part of keratoprostheses by Heparin coating.

### One-piece keratoprotheses

Cylindric polymer buttons of poly-Phenoxyethylacrylate (POEA) with 20 mm diameter and 5 mm height were obtained by thermal polymerization of Phenoxyethylacrylate in closed moulds with N,N-Azobisisobutyronitril (AIBN) as initiator and Ethyleneglycoledimethacrylate (EGDMA) as crosslinker.

Commercially available buttons of a copolymer of Laurylmethacrylate (LMA), Methylmethacrylate (MMA) and 2-Ethoxyethylmethacrylate (EOEMA) were obtained from Benz Research & Development Corporation, Sarasota, FL, USA (BENZ CLEAR HYDROPHOBIC HF-1 material).

Both polymers are hydrophobic acrylic polymers with a glass transition temperature of approximately 10°C. At the corneal temperature (- 35°C) both materials are flexible with their rigidity similar to corneal tissue.

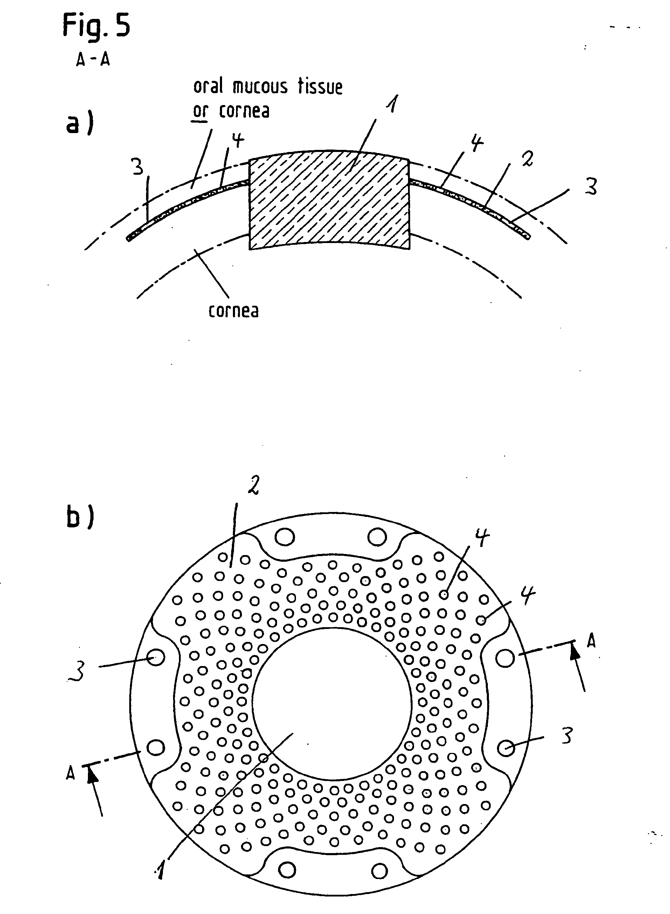

One-piece prototype keratoprostheses for epicorneal and endocorneal implantation were manufactured by cryo-milling and cryo-lathing from both materials with larger, more stable holes for suturing and smaller holes for tissue ingrowth (Figure 5 shows an example).

### Coating of POEA

The surface of POEA was activated (ionized) by treatment with Argon plasma.

In order to permanently render the outer surface of a keratoprosthesis (which is in contact with tearfilm) strongly hydrophilic, a solution of 2,3 Dihydroxypropyl-methacrylate (DHPMA) and UV initiator was sprayed on the activated POEA surface and polymerized by UV light. The DHPMA coated POEA surface was stable against light, hydrolysis and aging. It allowed perfect spreading of water.

For creating bioactive surfaces the activated POEA surface was coated by a layer-by-layer technique three times alternatively depositing Chitosan from crab shells (Chi) and Heparin sodium salt (Hep). The Chi-Hep-Chi-Hep-Chi-Hep coating resulted in a stable Heparin coating of the POEA.

In another experiment an additional layer of Fibroblast Growth Factor (FGF) was bound to the outer Heparin layer resulting in FGF coated POEA.

### Coating of HF-1

The surface of HF-1 polymer was activated (ionized) by treatment with Nitrogen plasma.

In order to permanently render the outer surface of a keratoprosthesis (which is in contact with tearfilm) strongly hydrophilic, a solution of 2,3 Dihydroxypropyl-methacrylate (DHPMA) and UV initiator was sprayed on the activated HF-1 surface and polymerized by UV light. The DHPMA coated HF-1 surface was stable against light, hydrolysis and aging. It allowed perfect spreading of water.

For creating bioactive surfaces the activated HF-1 surface was coated by a layer-by-layer technique two times alternatively depositing Chitosan from crab shells (Chi) and Heparin sodium salt (Hep). The Chi-Hep-Chi-Hep coating resulted in a stable Heparin coating of the HF-1 polymer.

In another experiment an additional layer of Fibronectin-like Engineered Protein Polymer (FEPP), a peptide known to promote cell adherence, was bound to the outer Heparin layer resulting in FEPP coated HF-1 polymer.

### Biological response to coated polymers

Coated and uncoated polymers were tested for adherence and proliferation of adherent corneal cells *in vitro.*

DHPMA coated POEA and HF-1 as well as uncoated HF-1 did not promote cell adherence or proliferation.

Heparin coated POEA and HF-1 resulted in poor cell adherence and proliferation.

Untreated POEA, FGF coated POEA and FEPP coated HF-1 polymer resulted in excellent cell adherence and proliferation.

A series of prototype keratoprostheses with geometric design similar to Figure 5 were manufactured from HF-1 material. The outer surface of the optic was coated with DHPMA, the sides of the optic and the haptic were coated with FEPP, and the inner surface of the optic was left untreated. These keratoprostheses were implanted in eight rabbit eyes. The rabbit cornea was trepanated to accept the optic of the keratoprosthesis, the keratoprosthesis was placed with the haptic on the cornea (epicorneal) and sutured to the cornea. Then the keratoprosthesis was covered with the rabbit's nictating membrane. After eight weeks observation time the inner and outer optic were completely clear without any fibrin adherence or retroprosthetic membrane formation, and the eyes did not show inflammatory responses.

The drawings serve for the explanation of the invention. The drawings show in
- Fig. 1 a-e: known examples of the keratoprosthesis;
- Fig. 2: a known keratoprosthesis;
- Fig. 3a,3b: optical cylinders of keratoprostheses;
- Fig. 4: an embodiment of an inventive keratoprosthesis in side view;
- Fig. 5a: a section view of the embodiment of Fig. 4 along the section line A-A in Fig. 5b; and
- Fig. 5b: a plan view of the embodiment of Fig. 4 and 5a.

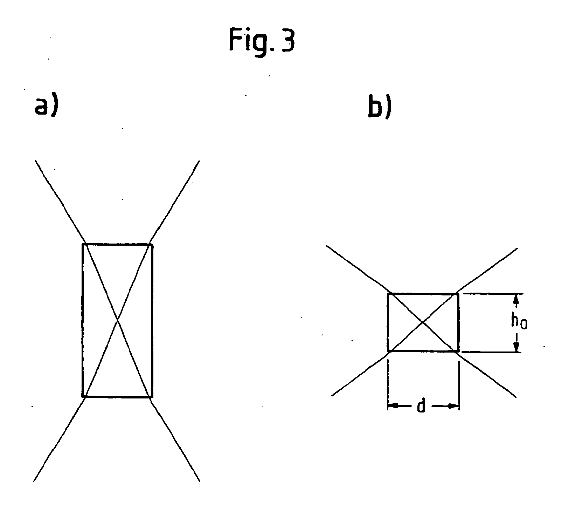

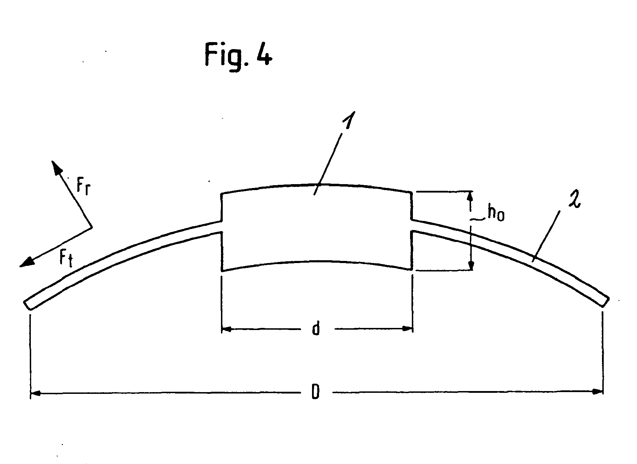

Out of the currently available keratoprostheses the Strampelli design has the best long-term clinical success, followed by the Pintucci design. Both keratoprosthesis designs require, however, a long optical cylinder (length hₒ) with a small diameter (d) in order to prevent the growth of retroprosthetic membranes (Fig. 3a). This means that both the iris and lens have to be removed and the patient will have a small visual field. Rendering the chemical nature of the posterior surface of the optical cylinder 1 such that the formation of a retroprosthetic membrane is prevented allows to significantly reduce the length hₒ of the optical cylinder 1 thus enlarging the visual field (Figure 3b) and allowing the patient's iris and lens to remain in place. Fig. 4 and 5 show a one-piece keratoprosthesis for epicorneal and endocorneal implantation larger more stable holes, for suturing and smaller holes, for tissue ingrowth.

The intraocular pressure exercised on the unsupported optical cylinder 1 of the keratoprosthesis is born by the haptic 2 attached to the remaining cornea. The force exercised by the intraocular pressure is proportional to the cross section of the optic, that means proportional to the square (d²) of the optic diameter (d). Therefore, the optic diameter shall be as small as reasonably possible, whereas the overall diameter (D) of the keratoprosthesis should be large in order to distribute the forces across a large haptic area (Figure 4 and 5).

The flexible haptic 2 will follow the movement of the surrounding corneal tissue and prevent local stress. The rigidity of the haptic material 15 similar to that of the corneal tissue, i.e. mimic the material. If the haptic material is too soft the haptic will exercise strong radial forces Fᵣ not evenly distributed over the corneal tissue and cause shear stress.

Preferrably both the optical cylinder 1 and the haptic 2 are made of the same flexible optically clear polymer. Alternatively the flexible haptic polymer can be polymerized as an interpenetrating network to the optic polymer which then may be rigid.

The front portion of the optical cylinder not in contact with corneal tissue can preferably be coated by a hydrophilic layer adsorbing water, enable a smooth gliding of the eye lid and support the spreading of artificial tear when instilled in the eye.

The haptic preferably enables the anchoring and ingrowth of surrounding tissue including vascularization. In order to enable this the topograghy of haptic surface can be textured with ridges, groves, pillars, cylindric holes, pores, mesh structure, spikes or similar, and the haptic form a scaffold to support tissue ingrowth. In order to maximize cellular attachment and minimize inflammatory response the surfaces of the keratoprosthesis in contact with tissue have a cell adhesive biochemical coating such as fibronecting and/or use the topography-associated surface free energy to promote cell adherence. The pores or holes 4 penetrate the haptic in order to allow vascularization.

## Claims

1. Keratoprosthesis having a central optical part (1) tightly connected to a peripheral haptic part (2) both made of hydrophobic polymers, where in the chemical nature of the surface oriented to the anterior chamber of the eye is such that it does not support fibrin adherence and membrane formation and the chemical nature of the textured haptic part (2) is configured to promote the adherence of cells and eventually the vascular ingrowth, **characterized in that**
- the optical part (1) has a cylindrical form and smooth surfaces,
- the surface of the optical part oriented toward the eyelid has been rendered hydrophilic, and
- the haptic material has a rigidity similar to that of the corneal tissue of the eye.

2. Keratoprosthesis according to claim 1, wherein the flexible haptic material is polymerized as an interpenetrating network to the optic material, which is a rigid material.

3. Keratoprosthesis according to claim 1 or 2, wherein it is a one-piece keratoprosthesis.

4. Keratoprosthesis according to any one of the claims 1 to 3, wherein the retroprosthetic surface of the optical part (1) is coated by Heparin.

5. Keratoprosthesis according to any of the one of the claims 1 to 4, wherein the keratoporosthesis material is an acrylic polymer with a glass transition temperature of approximately 10°C and having at the corneal temperature of about 35°C a rigidity similar to that of the cornea tissue.

6. Keratoprosthesis according to any one of the claims 1 to 5, wherein the keratoprosthesis material is POEA or a copolymer of LMA, MMA and EOEMA.

7. Keratoprosthesis according to any one of the claims 1 to 6, wherein the haptic part (2) is coated with FGF or FEPP.

## Patentansprüche

1. Keratoprothese, die einen zentralen optischen Teil (1) aufweist, der fest mit einem haptischen Randteil (2) verbunden ist, die beide aus hydrophoben Polymeren gefertigt sind, wobei die chemische Eigenart der der vorderen Augenkammer zugewandten Oberfläche so beschaffen ist, dass sie Faseranhaftung und Membranbildung nicht unterstützt, und die chemische Eigenart des strukturierten haptischen Teils (2) dafür konfiguriert ist, das Anhaften von Zellen und schließlich das Einwachsen von Gefäßen zu fördern,
**dadurch gekennzeichnet, dass**
- der optische Teil (1) eine zylindrische Form und glatte Oberflächen aufweist,
- die Oberfläche des optischen Teils, die dem Augenlid zugewandt ist, hydrophil gemacht worden ist, und
- das haptische Material eine Steifigkeit ähnlich derjenigen des Hornhautgewebes des Auges aufweist.

2. Keratoprothese nach Anspruch 1, wobei das flexible haptische Material als ein Netz polymerisiert ist, das das optische Material, das ein steifes Material ist, durchdringt.

3. Keratoprothese nach Anspruch 1 oder 2, die eine einteilige Keratoprothese ist.

4. Keratoprothese nach irgendeinem der Ansprüche 1 bis 3, wobei die retroprothetische Oberfläche des optischen Teils mit Heparin beschichtet ist.

5. Keratoprothese nach irgendeinem der Ansprüche 1 bis 4, wobei das Keratoprothesenmaterial ein Acrylpolymer mit einer Glasübergangstemperatur von ungefähr 10 °C ist und bei einer Hornhauttemperatur von etwa 35°C eine Steifigkeit ähnlich derjenigen des Hornhautgewebes aufweist.

6. Keratoprothese nach irgendeinem der Ansprüche 1 bis 5, wobei das Keratoprothesenmaterial POEA oder ein Copolymer von LMA, MMA und EOEMA ist.

7. Keratoprothese nach irgendeinem der Ansprüche 1 bis 6, wobei der haptische Teil (2) mit FGF oder FEPP beschichtet ist.

## Revendications

1. Kératoprothèse ayant une partie optique centrale (1) reliée de manière serrée à une partie haptique périphérique (2) toutes deux faites de polymères hydrophobes, dans laquelle la nature chimique de la surface orientée vers la chambre antérieure de l'oeil est telle qu'elle ne supporte pas l'adhérence fibrine et la formation de membrane et là nature chimique de la partie haptique texturée (2) est configurée pour favoriser l'adhérence de cellules et à terme la croissance vasculaire, **caractérisée en ce que**
- la partie optique (1) a une forme cylindrique et des surfaces lisses,
- la surface de la partie optique orientée vers la paupière a été rendue hydrophile, et
- le matériau haptique a une rigidité similaire à celle du tissu cornéen de l'oeil.

2. Kératoprothèse selon la revendication 1, dans lequel le matériau haptique flexible est polymérisé en tant que réseau interpénétrant dans le matériau optique, lequel est un matériau rigide.

3. Kératoprothèse selon la revendication 1 ou 2, dans laquelle la kératoprothèse est d'une seule pièce.

4. Kératoprothèse selon l'une quelconque des revendications 1 à 3, dans laquelle la surface rétroprothétique de la partie optique (1) est enduite avec de l'héparine.

5. Kératoprothèse selon l'une quelconque des revendications 1 à 4, dans laquelle le matériau de la kératoprothèse est un polymère acrylique ayant une température de transition vitreuse d'approximativement 10°C et ayant à la température cornéenne d'environ 35°C une rigidité similaire à celle du tissu cornéen.

6. Kératoprothèse selon l'une quelconque des revendications 1 à 5, dans laquelle le matériau de la kératoprothèse est du POEA ou un copolymère de LMA, MMA et EOEMA.

7. Kératoprothèse selon l'une quelconque des revendications 1 à 6, dans laquelle la partie haptique (2) est enduite de FGF ou FEPP.
